# EUROPEAN PATENT SPECIFICATION

(11) **EP 3 876 835 B1**
(45) Date of publication and mention of the grant of the patent: **26.11.2025**
(21) Application number: 19790491.5
(22) Date of filing: 17.10.2019
(51) Int. Cl.: A61B 5/053, A61B 5/00

(54) **MEDICAL DEVICES FOR ANALYZING EPITHELIAL BARRIER FUNCTION**
MEDIZINPRODUKTE ZUR ANALYSE DER EPITHELBARRIEREFUNKTION
DISPOSITIFS MÉDICAUX POUR ANALYSER UNE FONCTION DE BARRIÈRE ÉPITHÉLIALE

(30) Priority: 05.11.2018 SE 1851376
(43) Date of publication of application: 15.09.2021
(73) Proprietor: Scibase AB, 103 67 Stockholm (SE)
(72) Inventor: GRANT, Simon, 182 35 DANDERYD (SE); AKDIS, Cezmi, 7265 DAVOS WOLFGANG (CH); RINALDI, Arturo, 7270 DAVOS (CH)
(74) Representative: Valea AB
(86) International application number: PCT/EP2019/078227
(87) International publication number: WO 2020/094357

(56) References cited:
- WO-A1-2019/223874
- DE-A1- 102015 121 050
- US-A1- 2009 306 535
- US-A1- 2013 131 539
- US-A1- 2018 353 505
- JONES D M ET AL: "Modelling of epithelial tissue impedance measured using three different designs of probe", PHYSIOLOGICAL MEASUREMENT, INSTITUTE OF PHYSICS PUBLISHING, BRISTOL, GB, vol. 24, no. 2, 1 May 2003 (2003-05-01), pages 605 - 623, XP020073706, ISSN: 0967-3334, DOI: 10.1088/0967-3334/24/2/369
- STOLWIJK JUDITH A ET AL: "Impedance analysis of GPCR-mediated changes in endothelial barrier function: overview and fundamental considerations for stable and reproducible measurements", PFLUEGERS ARCHIV: EUROPEAN JOURNAL OF PHYSIOLOGY, SPRINGER VERLAG, BERLIN, DE, vol. 467, no. 10, 24 December 2014 (2014-12-24), pages 2193 - 2218, XP035544115, ISSN: 0031-6768, [retrieved on 20141224], DOI: 10.1007/S00424-014-1674-0
- WEGENER JOACHIM ET AL: "Experimental tools to monitor the dynamics of endothelial barrier function: a survey of in vitro approaches", CELL AND TISSUE RESEARCH, SPRINGER, DE, vol. 355, no. 3, 2 March 2014 (2014-03-02), pages 485 - 514, XP035331646, ISSN: 0302-766X, [retrieved on 20140302], DOI: 10.1007/S00441-014-1810-3

## Description

### Technical field

The present invention generally relates to the field of diagnosis of biological conditions and to medical devices and methods for non-invasively measuring electrical impedance spectroscopy in tissue of living subjects and for using the measured impedance in the diagnosis of biological conditions of the tissue. In particular, the present invention relates to medical devices and methods for analyzing and monitoring epithelial barrier function and barrier status using electrical impedance spectroscopy. Epithelial skin barrier may be determined for detecting drug effects and patient response to drug programs, screening purposes, e.g. screening infants on barrier defects, for diagnostic and treatment purposes of atopic dermatitis and for planning of preventive measures and preventive treatment in patients with barrier deficient skin.

### Background art

Epithelial tissues consist of layers of specialized cells closely bound together with a primary function to form a physical and chemical barrier between the body and the external environment. The epithelial barrier protects the internal tissues from environmental stresses, by minimizing water loss and preventing the entry of pathogens, pollutants, toxins and allergens through skin or mucosa (Ref. 1). Recent genome-wide association studies have shown that the role of barrier function of the epithelium is essential in several allergic diseases (Ref 2, 3). Barrier defects have been reported in atopic dermatitis, asthma, chronic rhino sinusitis, allergic rhinitis, eosinophilic esophagitis and colitis (Ref 4-9). This defect is a starting point of chronic inflammation and allergen sensitization and allows tissue-damaging factors to enter the deeper tissue and thus activate immune and inflammatory responses (Ref 10, 11).

Skin has two physical barrier structures: the stratum corneum and tight junctions (TJ)(Ref 10). The stratum corneum is the outermost layer of the epidermis, consisting of terminally differentiated keratinocytes, called corneocytes, which form a densely packed and extensively cross-linked lipid-protein matrix. The proteins filaggrin, loricrin and involucrin have a pivotal role in skin barrier function by interacting with keratin intermediate filaments (Ref 12). The most important component of the epithelial barrier is represented by the TJ that seal paracellular spaces at the very apical side in the mucosa and at the level of stratum granulosum in the skin between neighboring epithelial cells (Ref 13-15). TJ are responsible for the epithelial permeability, by controlling the paracellular flux of ions and bigger molecules, and physically separate the two different compartments. TJ are necessary for appropriate epithelial cell differentiation and function, with strong involvement in signal transduction and epithelial proliferation and differentiation (Ref 16-19). They form large complexes in the cell membrane consisting of three major types of transmembrane proteins: the claudin family, the tight junction associated MARVEL (MAL and related proteins for vesicle trafficking and membrane link) protein family, single-span proteins such as the immunoglobulin-like proteins junction adhesion molecules (JAM) and coxsackie and adenovirus receptor (CAR). Intracellularly, the transmembrane proteins bind to several scaffold proteins such as the zonula occludens family (ZO) and are consequently connected to the actin cytoskeleton (Ref 16, 20).

Historically, the epithelial barrier has been possible to assess in vitro, by measuring trans-epithelial electrical resistance (TEER), which represents the opposition of the epithelium to the passage of a steady electrical current. For this purpose, epithelial cells are cultured to an air liquid interface (ALI) in transwell plates. The confluence of the cellular integrity determines a sharp increase in TEER, indicating a low ion flux and a tight epithelial barrier; while the disruption of junctional complexes results in reduction of TEER. In addition, TEER measurements show good negative correlation with fluoresceinated dextran passage as demonstrated in ALI cultures of different tissues (Ref 24, 25).

In vivo there are few noninvasive methods to assess epithelial barrier function. One of these is the quantification of transepidermal water loss (TEWL) in the skin across the stratum corneum. Although TEWL increases in proportion to the level of damage, it is also affected by environmental factors such as humidity, temperature, season and moisture content of the skin. Other used noninvasive methods include stratum corneum hydration, colorimetry, skin surface pH, corneometetry and *sebometry* (U. Heinrich, U. Koop, et al.: Multicentre comparison of skin hydration in terms of physical-, physiological- and product-dependent parameters by the capacitance method (Corneometer CM 825), International Journal of Cosmetic Science, 2003, 25, 45-51). They provide information on different characteristics and/or condition of the skin, but they don't directly measure the barrier function (Ref 26).

Devices and methods for assessing the epithelial barrier function are known from US-2009/306535-A1, JONES D M ET AL: "Modelling of epithelial tissue impedance measured using three different designs of probe", PHYSIOLOGICAL MEASUREMENT, INSTITUTE OF PHYSICS PUBLISHING, vol. 24, no. 2, May 2003, ISSN: 0967-3334, DOI: 10.1088/0967-3334/24/2/369, US-2013/131539-A1, STOLWIJK JUDITH A ET AL: "Impedance analysis of GPCR-mediated changes in endothelial barrier function: overview and fundamental considerations for stable and reproducible measurements", PFLUEGERS ARCHIV: EUROPEAN JOURNAL OF PHYSIOLOGY, vol. 467, no. 10, 24 December 2014, ISSN: 0031-6768, DOI: 10.1007/S00424-014-1674-0, WEGENER JOACHIM ET AL: "Experimental tools to monitor the dynamics of endothelial barrier function: a survey of in vitro approaches", CELL AND TISSUE RESEARCH, vol. 355, no. 3, 2 March 2014, ISSN: 0302-766X, DOI: 10.1007/S00441-014-1810-3, DE-102015121050-A1, and WO-2019/223874-A1.

There is a need for improved and more accurate tools that can be used to analyze and assess epithelial skin barrier function in vivo that overcome the above mentioned disadvantages.

### Summary of the invention

An object of the present invention is to provide improved medical devices and methods for analyzing and assessing epithelial skin barrier in vivo.

A further object of the present invention is to provide improved medical devices and methods for detecting drug effects on patients and for determining patient response on epithelial skin barrier of drug delivery in vivo.

Yet another object of the present invention is to provide improved medical devices and methods for screening epithelial skin barrier function in vivo.

Another object of the present invention is to provide improved medical devices and methods for analyzing and assessing epithelial skin barrier in vivo, with an increased accuracy and reliability.

A further object of the present invention is to provide improved medical devices and methods for analyzing and assessing epithelial skin barrier in vivo that are stable against influence from environmental factors.

Yet another object of the present invention is to provide improved medical devices and methods alleviating and facilitating the measurement procedures and burden for both the patient and the user when analyzing and assessing epithelial skin barrier.

These and other objects of the present invention are achieved by a device according to claim 1. Further embodiments are defined in the dependent claims.

The present invention is based on a deeper understanding of dielectric properties of various tissues, which now makes it possible to establish a method to assess the epithelial barrier function in vivo, which is relatively stable against the influence of environmental factors. The inventive method can be used as a diagnostic instrument for skin inflammatory disorders with a barrier defect, such as atopic dermatitis (AD). The electrical impedance (EI) spectroscopy is a relatively new technique that previously has been used as means to characterize skin tumors. Electrical currents are transmitted through the skin at several depths and frequencies and the impedance response is measured, influenced by certain properties of tissue integrity. Generally, when there is an alteration in tissue structure and cellular composition, there is an imprinting in the electrical impedance spectrum related to the type of the tissue alteration (ref 27). In some diseases, such as melanoma, the measurements of tissue EI have been used for diagnosis, assessment of disease progression and evaluation of therapy. The inventors have now found that EI spectroscopy technique can be used in skin and mucosal diseases, where epithelial barrier dysfunction and the effects of certain treatments on epithelial barrier need to be monitored (Ref 4-7) and also "Electrical bioimpedance related to structural differences and reactions in skin and oral mucosa", Annals of the New York Academy of Sciences, 20 April 1999, Vol.873, pp.221-6, and Clinically normal atopic skin vs. non-atopic skin as seen through electrical impedance, Nicander, Ingrid; Ollmar, Stig, Skin Research and Technology, August 2004, Vol.10(3), pp.178-183.

Thus, according to the present invention, epithelial skin barrier function or skin barrier integrity can be evaluated/quantified. An impaired skin barrier is a pre-cursor to many disorders such as atopic dermatitis. Thus, skin barrier changes can be detected by means of the present invention and disorders such as atopic dermatitis can be predicted at an early stage e.g. on infants, youth, and adults. Further, the efficiency of various treatments of such diseases can be assessed. Quantifying the degree of sensitivity for allergens and toxic/irritant substances, both on skin, oral cavity,
bronchial, esophageal, stomach, duodenum, small and large intestine, vagina and urogenital system are further applications of the present invention. Monitoring treatment of skin diseases such as psoriasis (in addition to eczema) and assessing lesions such as lichen in the skin oral cavity are yet other conceivable applications of the present invention. In addition, the present invention can be used to assess periodontitis to e.g. quantify risk of loss of teeth.

EI spectroscopy may further be used for the prediction of AD in infants, allowing the identification of the risk of the disorder and thus the possibility to apply preventive measures. Furthermore, EI spectroscopy can be used for follow-up of cutaneous lesions to obtain information on the effect of a topical or systemic treatment and gather additional information for monitoring the stage and severity of a lesion. Moreover, it may also be a useful non-invasive, cost-effective tool for the overall clinical assessment for the follow-up of a patient, without performing sophisticated assays for barrier evaluation, such as the analyses of filaggrin mutations from DNA. As a follow-up of these initial data in different mouse models, EI spectroscopy remains to be further studied in human subjects affected by AD and/or other skin inflammatory disorders. Measurements performed in both lesional and non-lesional skin can be compared in order to detect any difference in skin permeability and electrical response due to the inflammatory state. In addition, a comparison of EI measurements in AD patients and in healthy volunteers will indicate whether patients have an appreciable and detectable defect in their skin electrical behaviour.

There is disclosed a method for assessing and monitoring epithelial barrier function in vivo of a subject using EI measurements. The method comprises initiating an impedance measurement session including passing an electrical current through the skin of the subject to obtain values of skin impedance of a target tissue region, said data comprising at least one impedance value measured in the target tissue region at different tissue layers. Further, an evaluation procedure is applied for analyzing the epithelial barrier function in the target tissue region based on the measured data set of impedance values for the target tissue region at different tissue layers. The procedure evaluates the obtained data set of impedance values to provide an outcome indicating a status of the epithelial barrier function of the subject.

There is further disclosed a method for determining patient response in vivo to a drug using electrical impedance measurements. The method comprises initiating an impedance measurement session including passing an electrical current through the skin of the subject to obtain values of skin impedance of a target tissue region, said data comprising at least one impedance value measured in the target tissue region at different tissue layers. Further, an evaluation procedure is applied for analyzing the epithelial barrier function in the target tissue region based on the measured data set of impedance values for the target tissue region at different tissue layers. The obtained data sets of impedance values are evaluated to provide an outcome indicating a status of the epithelial barrier function of the subject. The patient response to the drug is based or determined on status of epithelial barrier function and clinical data including drug prescription.

There is further disclosed a method for screening epithelial barrier function of a number of subjects in vivo using electrical impedance measurements. The method comprises performing impedance measurement sessions of subjects including passing an electrical current through the skin of each subject to obtain values of skin impedance of a target tissue region, said data comprising at least one impedance value measured in the target tissue region at different tissue layers for each subject. An evaluation procedure for analyzing the epithelial barrier function in the target tissue region based on the measured data set of impedance values for the target tissue region at different tissue layers for each subject is applied. Further, the obtained data sets of impedance values are evaluated to provide an outcome indicating a status of the epithelial barrier function of each subject.

According to the present invention, there is provided a medical device for assessing and monitoring epithelial barrier function of subject in vivo according to claim 1. The device comprises an impedance measuring unit configured to pass an electrical current through the skin of the subject to obtain values of skin impedance of a target tissue region, said data comprising at least one impedance value measured in the target tissue region at different tissue layers, an evaluation unit configured to apply an evaluation procedure for analyzing the epithelial barrier function in the target tissue region on the basis of the measured data set of impedance values for the target tissue region at different tissue layers and to evaluate the obtained data set of impedance values to provide an outcome indicating a status of the epithelial barrier function of the subject. The medical device according to the present invention can preferably be used for the method disclosed.

In embodiments of the present invention, the medical device includes a probe for measuring electrical impedance of tissue of a subject. The probe comprises a plurality of electrodes, the electrodes being adapted to be placed in direct contact with the skin of the subject and being connectable to an impedance measuring circuit adapted to apply a voltage and to measure a resulting current to determine an impedance signal. In preferred embodiments, the probe further comprises a switching circuit for selectively activate electrode pairs by connecting at least two of electrodes with the impedance measuring circuit and disconnecting the remaining electrodes from the impedance circuit, wherein the voltage is applied at the two electrodes and the resulting current is measured between at least two electrodes. The switching circuit is adapted to receive control signals instructing the switching circuit to activate electrode pairs in accordance with a predetermined activation scheme, the predetermined activation scheme including to activate adjacent electrodes in a successive manner to gradually scan tissue of the subject at a first tissue depth so as to obtain a sequence of impedance signals from a selected tissue depth.

According to embodiments, the probe is provided with electrodes that have an elongated rectangular shape and are arranged at the probe in parallel rows. However, there are a number of alternative designs. For example, the electrodes may be arranged as concentric rings, or as squares. The electrodes may be arranged with micro-spikes wherein each electrode comprises at least one spike. The spikes are laterally spaced apart from each other and having a length being sufficient to penetrate at least into and/or through the stratum corneum in case of skin measurements. In an alternative embodiment, the electrodes are non-invasive and each electrode has a substantially flat surface adapted to be placed against the tissue of the subject. It is also possible to combine electrodes provided with micro-spikes with non-invasive electrodes.

WO 01/52731 discloses example medical electrodes for sensing electric bio-potentials created within the body of a living subject. The electrode comprises a number of micro-spikes adapted to penetrate the skin. The micro-spikes are long enough to reach the stratum corneum and penetrate at least into the stratum corneum and are electrically conductive on their surface and connected to each other to form an array. In EP 1 437 091, an apparatus for diagnosis of biological conditions using impedance measurements of organic and biological material is disclosed. The apparatus comprises a probe including a plurality of electrodes, where each electrode is provided with a number of micro-spikes each having a length being sufficient to penetrate at least into stratum corneum. The micro-spikes according to EP 1 437 091 are also "nail-like", i.e. they have stem having a substantially circular cross-section with a constant or a gradually decreasing diameter and a tip-portion with a substantially spherical or spike-shaped tip.

In embodiments of the present invention, the probe may have a spherical shape, i.e. the surface of the probe provided with electrodes is spherically shaped.

According to embodiments of the present invention, the methods and medical devices can be used for moisturizer therapy selection where EIS measurements is used to determine the most suitable moisturizer for an individual (usually AD) patient.

According to embodiments of the present invention, EIS monitoring of an area that flares can be used to predict and prophylactically treat with corticosteroids or moisturizers etc.

According to embodiments of the present invention, the methods and medical devices can be used to determine appropriate timing to cease topical corticosteroid use.

According to embodiments of the present invention, the methods and medical devices can be used to determine the extent of subclinical spread of inflammation outwards from a eczema flare/rash.

According to embodiments of the present invention, the methods and medical devices can be used to identify infants with degraded barrier function and/or inflammation before the development of AD symptoms.

As the skilled person realizes, steps of the methods according to the present invention, as well as preferred embodiments thereof, are suitable to realize as computer program or as a computer readable medium.

Generally, all terms used in the claims and description are to be interpreted according to their ordinary meaning in the technical field, unless explicitly defined otherwise herein. All references to "a/an/the [element, device, component, unit, means, step, etc.]" are to be interpreted openly as referring to at least one instance of the element, device, component, unit, means, step, etc., unless explicitly stated otherwise. The steps of any method disclosed herein do not have to be performed in the exact order disclosed unless explicitly defined otherwise herein.

Further objects and advantages of the present invention will be discussed below by means of exemplifying embodiments.

### Brief description of the drawings

Exemplifying embodiments of the invention will be described below with reference to the accompanying drawings, in which:
Fig. 1 is a schematic block diagram of one embodiment of a medical device according to the present invention;
Fig. 2a - c: Epicutaneous administration of increasing concentrations of papain damages the epithelial barrier, causing a dose-dependent decrease of EI and increase of TEWL of skin;
Fig. 3a - c: Papain downregulates the expression of molecules involved in the epithelial barrier function;
Fig. 4a - c: Trypsin shows a similar effect on skin epithelial barrier, causing a decrease of EI and an increase of TEWL;
Fig. 5a - b: Tape stripping leads to a reduction of EI and an increase of TEWL of the skin.
Fig. 6: Cholera toxin decreases EI of skin ex vivo.
Fig. 7: Electrical impedance measured at the superficial layer shows a stronger decrease when compared to the deeper layer after papain treatment and tape stripping.
Fig. 8: Nyquist plot showing the effect of papain on the EI represented as a vector on a Nyquist Plot where the two components of the EI, the real part and the imaginary part, are plotted on the X and Y axes.
Fig. 9 AD patients show significantly decreased EI values compared to healthy subjects and their lesions are characterized by a decrease of EI and an increase of TEWL, if compared to non-lesional skin.
Fig. 10 Lesional skin shows an increase of EI and a decrease of TEWL during the 21 day treatment.

### Description of exemplifying embodiments

The following is a description of exemplifying embodiments in accordance with the present invention. This description is not to be taken in limiting sense, but is made merely for the purposes of describing the general principles of the invention. Even though particular types of probes including micro-invasive as well as non-invasive will be described, the invention is also applicable to other types of such as invasive probes.

Thus, preferred embodiments of the present invention will now be described for the purpose of exemplification with reference to the accompanying drawings, wherein like numerals indicate the same elements throughout the views. It should be understood that the present invention encompasses other exemplary embodiments that comprise combinations of features as described in the following. Additionally, other exemplary embodiments of the present invention are defined in the appended claims.

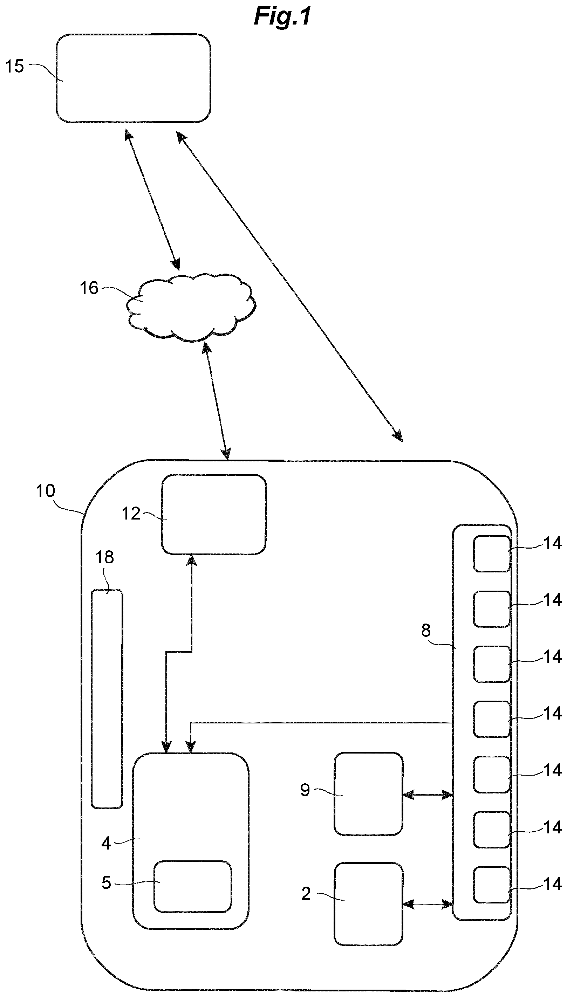

Referring first to Fig. 1, a general description of a medical device according to the present invention will be discussed. The device 10 comprises an impedance measuring circuit or unit 2 and a analyzing or evaluation unit 4 for analyzing the epithelial barrier function in the target tissue region on the basis of the measured data set of impedance values for the target tissue region at different tissue layers and evaluating the obtained data set of impedance values to provide an outcome indicating a status of the epithelial barrier function of the subject. The impedance measuring unit 2 is adapted to obtain impedance data of a target tissue region of the tissue of the subject. It is to be understood that the impedance data of the target tissue region comprises at least one impedance value obtained at different tissue depths (or layers), for example, at four different depths, and over a spectrum of frequencies, for example, at 35 different frequencies from 1 kHz to 2.5 MHz.

The tissue impedance measurement for obtaining the impedance data of the target tissue region may be performed by means of a probe 8 integrated in the medical device 10 or a probe being external to the medical device 10 and connected to the medical device 10. Irrespective of being external or integrated, the probe may comprise a plurality of electrodes 14 adapted to be placed in contact with the tissue to be analyzed, typically skin of the subject. The tissue impedance may be measured by applying an AC voltage over a pair of electrodes and measure the resulting current passing the same pair of electrodes. In embodiments, 2-point measurements are used by applying voltage and measure current over one pair of electrodes. The remaining electrodes may be grounded or free floating. In embodiments of the present invention, the probe 8 comprises, for example, seven or five electrodes, e.g. shaped as rectangular electrode bars. The electrodes are adapted to be placed in direct contact with the skin.

In an embodiment, adjacent electrodes are separated with a distance of about 0.3 mm and having a length of about 5 mm, has shown to be a practical and useful configuration for detections of diseased conditions such as malignant melanoma, both with regard to spatial resolution in a lateral dimension and in a depth dimension. A skin area of about 5x5 mm or about 25 mm² is thus covered by the probe and at high frequencies, above about 100 kHz, the deepest tissue layer being reached is about 2.5 mm which has been proven to be a clinical relevant depth. In order to cover a larger skin area, the probe can be moved to a neighboring skin site. However, as the skilled person realizes, the probe may include more or less than seven electrodes, for example 3, 4, 5 or 9 electrodes. Further, other electrode dimensions, geometries and other spacing between adjacent electrodes are conceivable, for example, electrodes having a width of about 4 mm and a length of about 8 mm.

By selecting adjacent pairs of electrodes, the topmost layer of the skin can be scanned in steps, and by selecting pairs that are spaced further apart, i.e. electrode pairs with one or more intermediate electrodes, the resulting current path allows for measurement at deeper skin layers. The possibility to measure inter alia the topmost skin layer in small (determined inter alia by the spacing between adjacent electrodes and the frequency of the applied current) consecutive partitions is important since it allows for detection of small anomalies in the skin and tissue. Each electrode of the probe may be set in four different states including inject (the electrode is set to inject measurement current into the tissue), measure (the resulting current from the tissue is measured via the electrode), ground (the electrode is grounded to prevent leakage of superficial current when measurements are performed using other electrodes) and floating (the electrode is disconnected).

The evaluation unit 4 may include storage units (not shown) for storing, for example, obtained impedance data performed on the patient. The diagnosing unit 4 may also include a processing circuit 5, in this embodiment included in the diagnosing unit 4, adapted to process obtained impedance data to reduce the number of variables by removing insignificant variables by performing linear or non-linear projections of the impedance data to lower subspaces. In preferred embodiments of the present invention, principal component analysis (PCA) is used. An alternative approach is to use parallel factor analysis (PARAFAC). Further, classification rules determined by means of, for example, linear discriminant analysis (LDA) or soft independent modelling of class analogy (SIMCA) may be used to improve the evaluation.

Moreover, the evaluation unit 4 may communicate with display means for displaying, for example, an epithelial skin barrier status. The evaluation unit 4 applies an evaluation procedure for analyzing the epithelial barrier function in the target tissue region on the basis of the measured data set of impedance values for the target tissue region at different tissue layers and evaluates the obtained data set of impedance values to provide an outcome indicating a status of the epithelial barrier function of the subject. A magnitude of the measure impedance may be determined and reduction or decrease of the magnitude indicates an impaired or decreased epithelial barrier function of the subject. Reference data and/or clinical data may be used in the evaluation. According to the present invention, the evaluation unit 4 uses a trained evaluation procedure for analysis of the measured data set of impedance values, wherein the trained evaluation procedure extracts impedance data from the impedance spectra from obtained data sets of impedance values reflecting tissue characteristics of epithelial barrier function and evaluates the obtained data set of impedance to provide the outcome indicating a status of the epithelial barrier function of the subject.

According to embodiments of the present invention, each electrode is provided with spikes, thereby forming a spiked surface. As has been discussed above, the probe, in preferred embodiments, may include five rectangular areas or bars. In this configuration, each bar contains an array of, for example, 45 (15 x 3) or, 57 (19 x 3) micro-spikes. Each bar is about 0.75 mm wide and 5 mm long. The distance between adjacent bars is about 0.2-0.5 mm. The active part of the probe is thus about 5 x 5 mm. Each micro-spike has a length of approximately 100 micrometer, as measured from its base, and a thickness of at least 20 micrometer. The electrode bars and micro-spikes can be made of plastic material in a moulding process. The material could be made intrinsically conductive or covered with a conductive layer such as gold. In an alternative embodiment, the electrode bars and micro-spikes are made of, for example, plastic or silicon and covered with metal, for example, gold having a thickness of at least 1 micrometer. However, other materials comprising a conductive surface with similar dimensions would work, but it should be selected to be biocompatible. In, for example, the patent applications EP 1959828, EP 1600104, and EP 1437091 by the same applicant, different probe concepts having such micro-spikes are described.

In another embodiment, the electrode bars are non-invasive and substantially flat. In, for example, US 5,353,802 by the same applicant, a probe concept including non-invasive electrodes has been described.

In other embodiments of the present invention, the probe is spherically shaped, i.e. the surface including the electrodes that is pressed against the skin or tissue during a measurement has a spherical shape. This also means that the electrodes may be at least partly spherically shaped.

For example, each spike may have a length of 0.01 to 1 mm. The spikes may be arranged on electrodes, in turn arranged on the probe, where each electrode may comprise from at least two spikes to about 100-200 spikes in certain applications, and any number in between. In the patent US 9,636,035 by the same applicant, examples of preferred embodiments of spike designs are described. By such configurations of spikes an increased versatility and increased adaptability in terms of capacity requirements can be achieved, in addition to possibly alleviating the problem of non-linear effects of the stratum corneum.

A control circuit 9 may be configured to control, for example, switching cycles/sequences of the electrodes 14 in accordance with a predetermined activation procedure or scheme. This predetermined activation scheme may include an activation of adjacent electrode in a successive manner to gradually scan tissue of the subject at a first tissue depth, which scanned tissue depends to a large extent on spacing between activated electrode pairs so as to obtain a matrix of impedance signals from different tissue depths.

The evaluation unit 4 is configured to pre-process the impedance data, for example, reduction of noise content and/or reduction of the dimensionality. The noise reduction may include reduction of noise in the impedance magnitude and/or phase angle spectra. The noise reduction may for example be made with the use of a Savitsky-Golay smoothing filter. Data on the subject's physical conditions may also be utilized by the diagnosing unit 4 and the data on the physical conditions may be parameterized and further used in the diagnosing process. Further, the pre-processing may comprise detection and correction of spikes or other artefacts, enabling removal of spikes or artefacts in the impedance spectrum, i.e. magnitude and/or phase angle spectra. Spikes may for example be detected with a median filter with an adequate window size. Data points of the filtered data that differ too much from raw data may be considered to be a spike or other artefact and may be corrected by e.g. linear interpolation.

The evaluation unit 4 may further comprise a pre-filter enabling rejection of measurement that do not fulfill one or a few specific criteria, such as cut-offs. The pre-filter may be applied on impedance data that has been corrected/adjusted e.g. by pre-processing as discussed above. For example, the magnitude values and/or phase angle values may all be required to fall within a specified magnitude range of a specified phase range, respectively, in order for a measurement not to be rejected. If the measurement is on a human/animal skin, the criteria, such as the ranges, may be set such as non-physiological measurements are rejected. Also, a specific criteria may be set for a certain value relating to a specific frequency.

The evaluation unit 4 may further include a classifier to assess whether quality of measured impedance data is good. This procedure may be combined with pre-processing and/or pre-filtering to further improve quality of the data. Examples of such classification include assessment of the variation, e.g. the variance or standard deviation, of magnitude and/or phase angle in different permutations at one or a plurality of frequencies. Further examples encompass the absolute values of magnitude and/or phase angles that may be studied, for example the median value or average value, skewness of magnitude, derivative of magnitude or phase angle, or phase angle.

The medical device 10 may further include a communication unit 12 capable of transmitting/receiving data to/from external units 15, such as a laptop computer, a handheld computer/device, a computer embedded into the device, a database, a cloud-based arrangement, etc., directly with the unit or network itself or via a wireless network 16. In this way, the device 10 may be supplied with, for example, clinical data for use in the evaluation. Moreover, data obtained with the medical device 10 such as impedance data from measurements can also be downloaded to external devices 15 via the communication unit 12.

Furthermore, the medical device 10 includes a pressure applying unit 18 configured to apply a predetermined pressure on the tissue or skin when activated and the probe 8 is pressed against the tissue or skin during the measurement. Preferably, the pressure is constant during the measurement session. For example, a pressure in a range of 1 - 12 N may be applied, or in preferred embodiments a pressure in a range of 3 - 10 N, or in further preferred embodiments in a range of 5 - 7 N or as in a certain embodiments in a range of 5.5 - 6.5 N. In embodiments of the present invention, the applied predetermined pressure may be combined with or replaced by a sucking action, which thus attaches the probe to the tissue or skin during the measurement.

It is to be understood that in the context of the present invention and in relation to electrical components electrically connected to each other, the term connected is not limited to mean directly connected, but also encompasses functional connections having intermediate components. For example, on one hand, if an output of a first component is connected to an input of a second component, this comprises a direct connection. On the other hand, if an electrical conductor directly supplies a signal from the output of the first component substantially unchanged to the input of the second component, alternatively via one or more additional components, the first and second components are also connected. However, the connection is functional in the sense that a gradual or sudden change in the signal from the output of the first component results in a corresponding or modified change in the signal that is input to the second component.

Although exemplary embodiments of the present invention has been shown and described, it will be apparent to those having ordinary skill in the art that a number of changes, modifications, or alterations to the inventions as described herein may be made. Thus, it is to be understood that the above description of the invention and the accompanying drawings is to be regarded as a non-limiting example thereof and that the scope of protection is defined by the appended patent claims.

### Test results

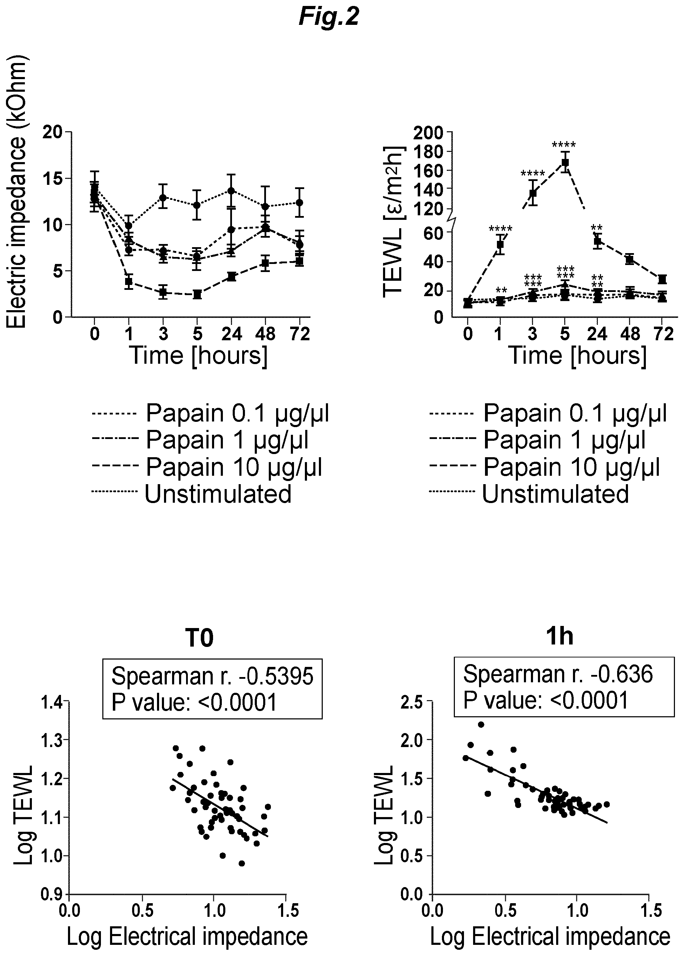

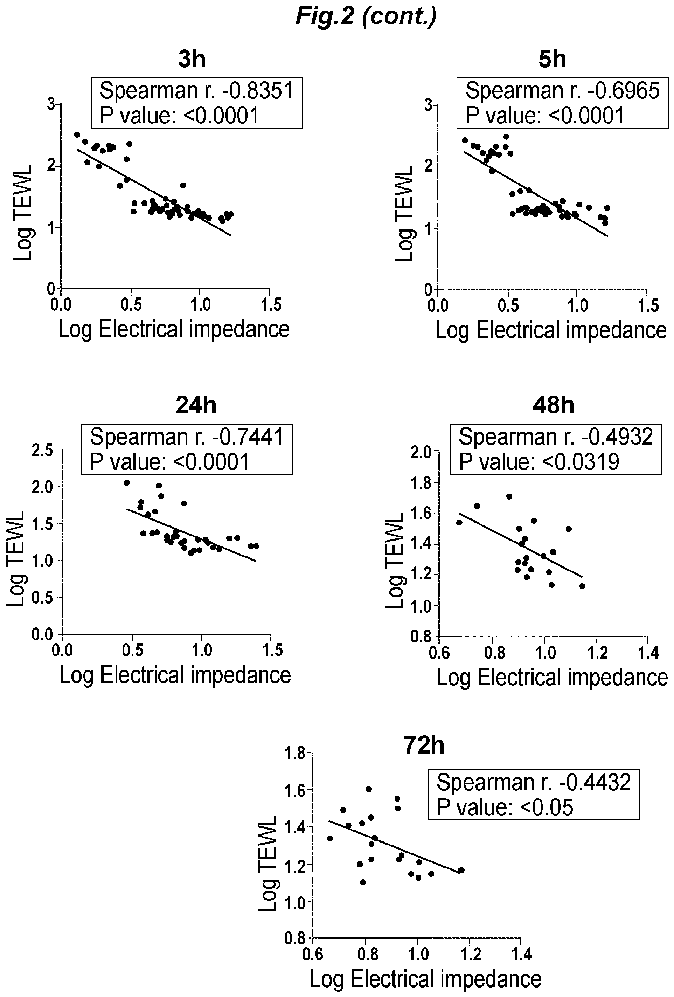

Hence, the inventors have found that EI spectroscopy can be used for the in vivo detection of the epithelial barrier function. As shown in Figure 2 a dose-dependent reduction of EI was detected as early as 1 hour after the treatment, reflecting the decreased epithelial barrier function.

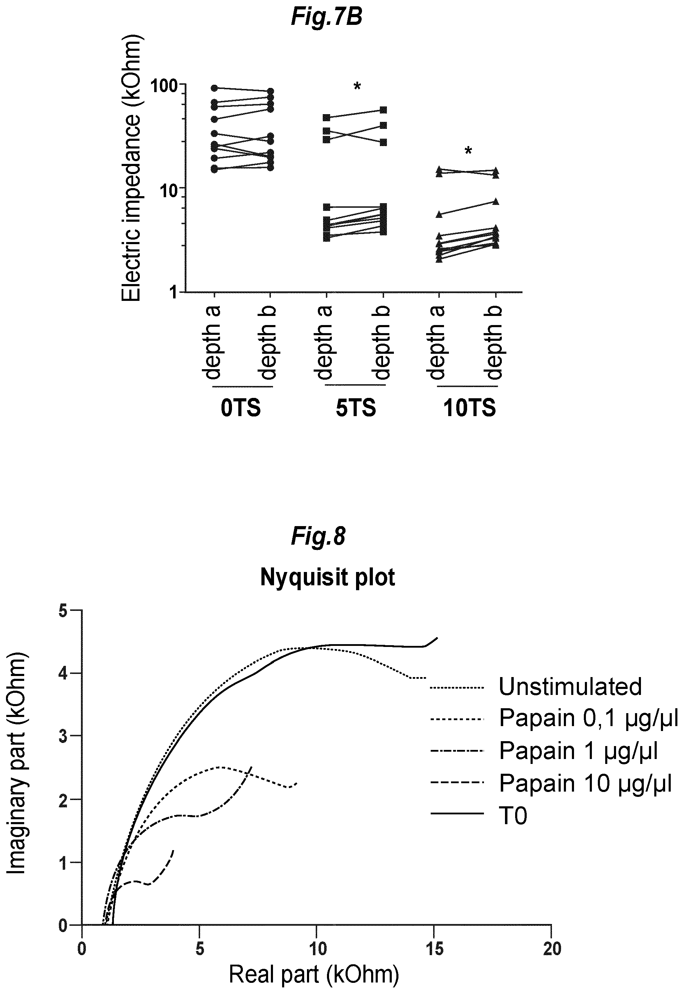

EI spectroscopy determinations show a clear negative correlation with another biomarker of epithelial barrier damage in the skin, which is transepidermal water loss (TEWL). The increase in TEWL demonstrates barrier damage which is in parallel to decrease in EI spectroscopy that also demonstrates barrier damage. In addition, to show clearly the effect of papain on the EI, we represented it as a vector on a Nyquist Plot, by plotting the two components of the EI, the real part and the imaginary part, on the X and Y axes, see Fig. 8. We clearly observed that at 5 hours from the papain application, the curves obtained were significantly different from the ones obtained in the control mice, in which only PBS was epicutaneously applied.

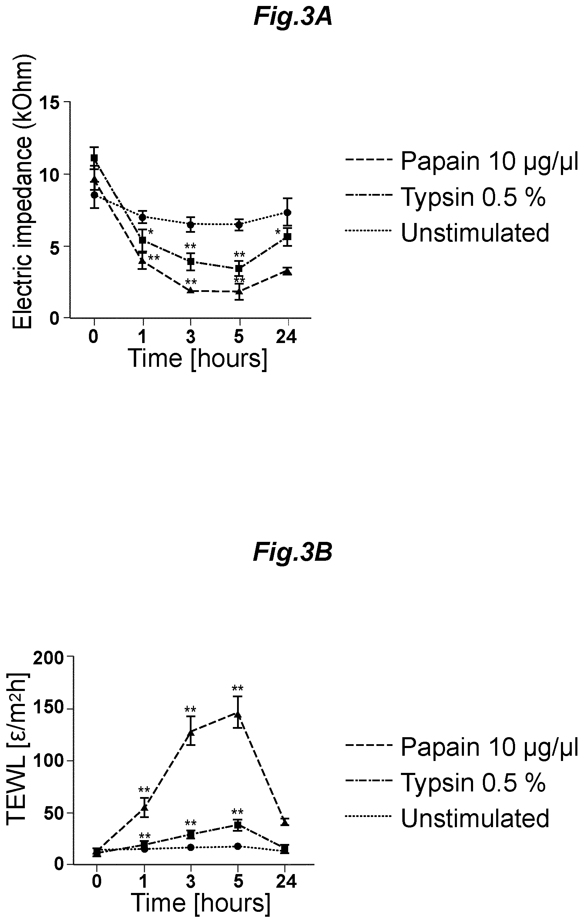

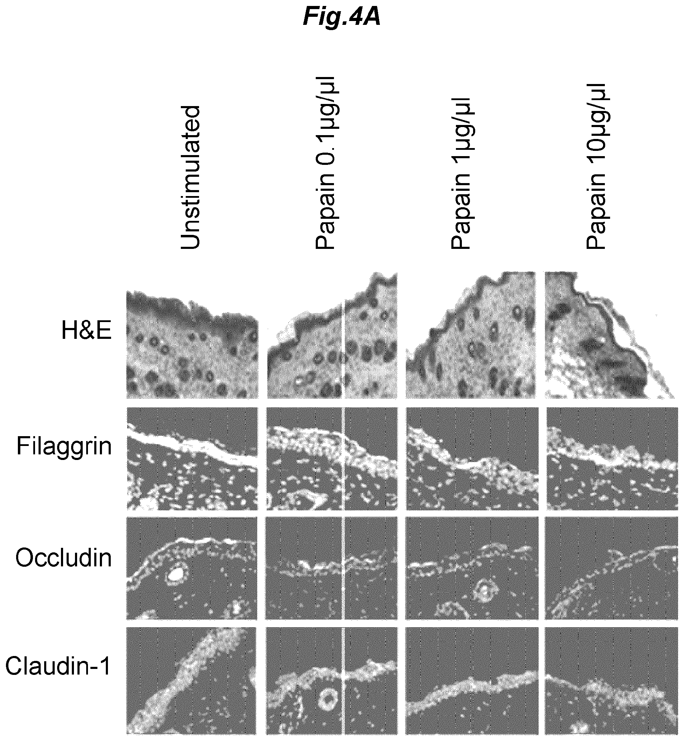

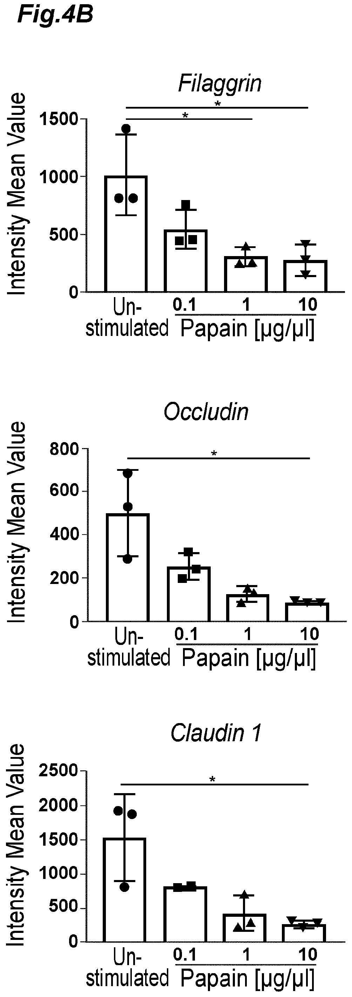

Barrier disruption induced by papain was confirmed by histological analysis, which showed an impaired stratum corneum and higher cellular infiltration after papain application, Fig. 3a. In addition, we checked the expression of molecules important for the skin barrier function, such as Filaggrin or the TJ molecules occludin and claudin-1 by immunofluorescence staining. Downregulation of the expression of all three barrier molecules was observed in a dose-dependent manner, Fig. 4a and 4b.

EI spectroscopy detects epithelial barrier as it decreases together with the damage of epithelial barrier molecules as demonstrated by decreased mRNA expressions of filaggrin, loricrin and involucrin, demonstrating overall impairment of the stratum corneum barrier function. These results confirmed the impairment of the skin barrier function by papain treatment and its demonstration by EI spectroscopy.

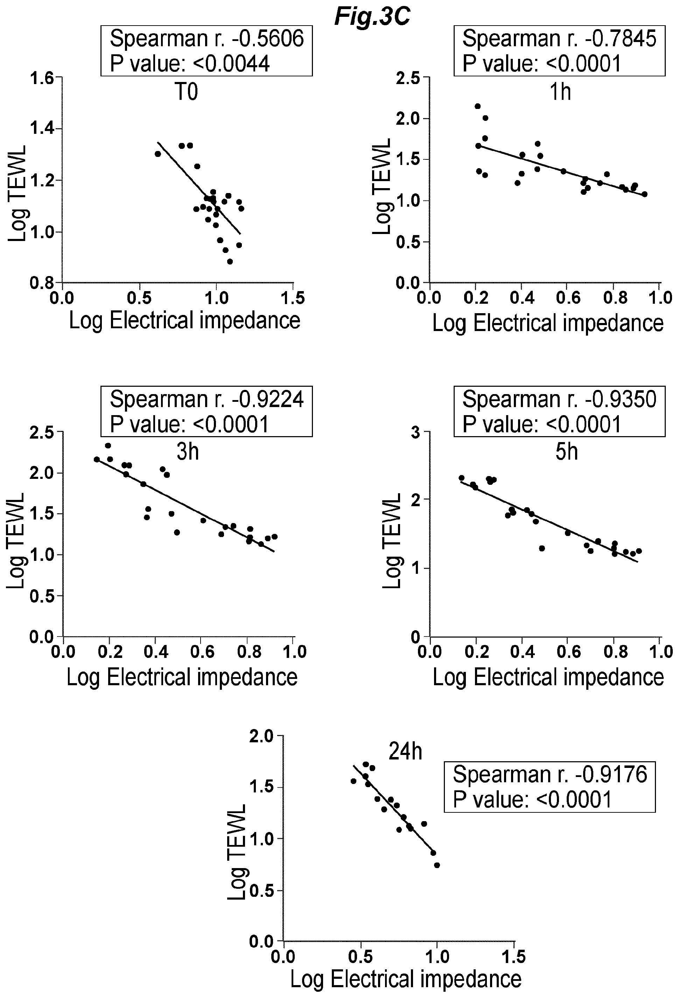

The effect of another protease, the serine protease trypsin, which was applied epicutaneously to mice skin by following the same protocol used for the papain application. As shown in Fig. 3, a significant decrease of EI and a simultaneous significant increase of TEWL after trypsin treatment, consistent with the data in papain exposure. Similarly, EI and TEWL values showed significant inverse correlation.

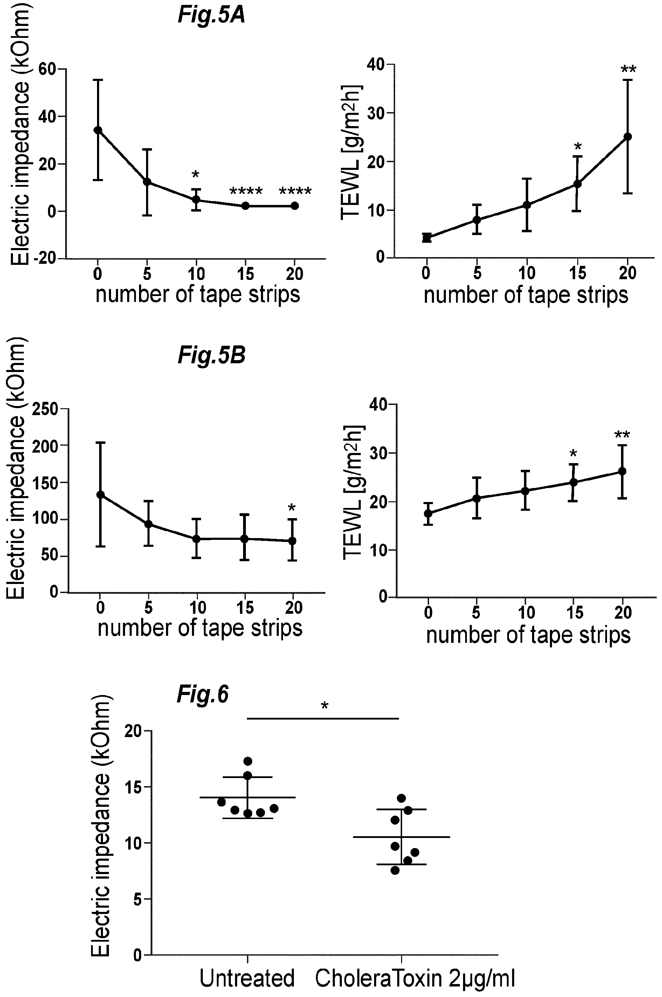

To further investigate the accuracy of epithelial barrier detection, we tested the EI and TEWL methods on nude mice skin after damaging the epithelial barrier by tape stripping, a simple and efficient method through which the cell layers of the stratum corneum are successively removed by using adhesive films. EI and TEWL measurements have been performed before tape stripping and immediately after 5, 10, 15 and 20 tape strips. We detected a significant reduction of EI after tape stripping, reflecting the decreased epithelial barrier function. In parallel measurements, we observed an increase of TEWL, confirming that the skin surface barrier function was reduced, Fig. 5a. The same protocol was followed in human healthy volunteers, in whom results obtained in mice were confirmed with a reduction of EI and an increase of TEWL observed after tape stripping Fig. 5b.

Epithelial barrier damage induced by cholera toxin is detected by EI spectroscopy. Back skin from C57BL/6 nude mice was isolated and incubated at 37°C for 1h in presence of 2 µg/ml cholera toxin. We observed that after one hour cholera toxin treatment, the EI was significantly reduced, in comparison to the control condition with PBS treatment, Fig. 6. Once again, our observations indicate the validity of EI spectroscopy as a method to detect epithelial barrier function.

The data demonstrates that EI spectroscopy can be a direct method to assess the skin epithelial barrier function in vivo. Based on our results, EI spectroscopy represents a good candidate approach for the study of and characterization of skin inflammatory disorders, such as AD. AD affects up to 20% of children and up to 4.9% of adults (Ref 21, 22). The hallmark features are itch and eczematous skin lesions that manifest often in early infancy with a course of remissions and exacerbations. An impaired epidermal barrier, characterized both by defective filaggrin protein expression and TJ defects, has been described (Ref 23). EI spectroscopy facilitate the early diagnosis of AD in infants, allowing the identification of the risk of the disorder and thus the possibility to apply preventive measures. Furthermore, EI spectroscopy can be used for follow-up of cutaneous lesions to obtain information on the effect of a topical or systemic treatment and gather additional information for monitoring the stage and severity of a lesion. Moreover, it may also be a useful non-invasive, cost-effective tool for the overall clinical assessment for the follow-up of a patient, without performing sophisticated assays for barrier evaluation, such as the analyses of filaggrin mutations from DNA.

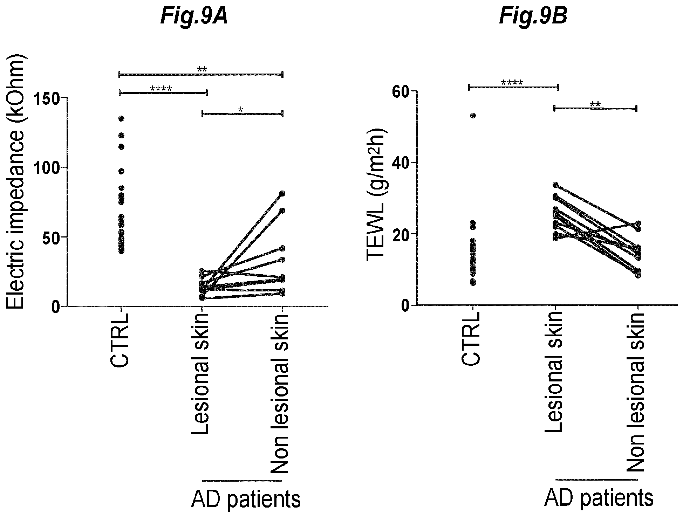

Fig. 9 illustrates test results where AD patients show significantly decreased EI values compared to healthy subjects and their lesions are characterized by a decrease of EI and an increase of TEWL, if compared to non-lesional skin. EI (a) and TEWL (b) were measured in healthy controls and AD patients in the same body site. In patients measurements were performed both on a lesion and on a non-lesional area close to that lesion. EI is expressed in kOhm, at the frequency of 1000 Hz. TEWL is expressed in g/m2h. *: p<0.05, **: p<0.01,, ****: p<0.0001.

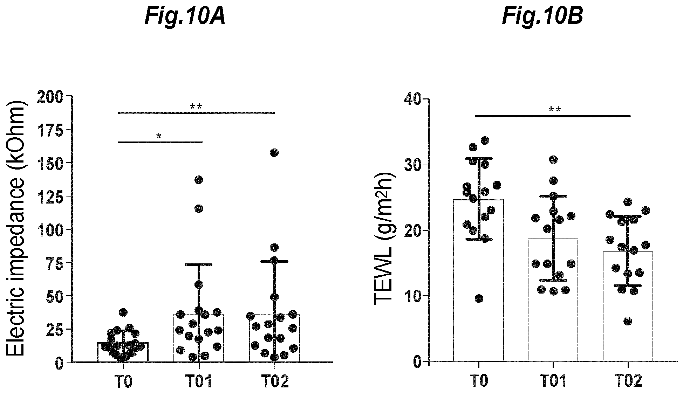

Fig. 10 illustrates test results where lesional skin shows an increase of EI and a decrease of TEWL during the 21 days treatment. T0 relates to the measurement at the first visit at the clinic, T01 is a the visit in the middle of the treatment and T02 the final visit. EI (a) and TEWL (b) were measured in AD patients in the same body site over a time of 21 days, which corresponds to the duration of the treatment at the clinic. EI is expressed in kOhm, at the frequency of 1000 Hz. TEWL is expressed in g/m2h. *: p<0.05, **: p<0.01.

### Description of test result figures

Fig. 2: **Epicutaneous administration of increasing concentrations of papain damages the epithelial barrier, causing a dose-dependent decrease of EI and increase of TEWL of skin.** WT C57BL/6 mice were depilated on their back by using a depilatory cream. Three days after depilation 100 µl of a solution containing different doses of the protease papain (0.1µg/µl, 1µg/µl and 10µg/µl), or PBS as control (unstimulated), by means of a skin patch. EI (a) and TEWL (b) measurements were performed before treatment and 1, 3, 5, 24, 48, 72 hours after the treatment. (c) Spearmen correlation between EI and TEWL is shown for each time point. Electrical impedance is expressed in kOhm, at the frequency of 1000 Hz. TEWL is expressed in g/m2h. Data are shown as mean ± SD (n=8). *: p<0.05, **: p<0.01, ***: p<0.001, ****: p<0.0001
Fig. 3: **Papain downregulates the expression of molecules involved in the epithelial barrier function. (a)** Hematoxylin and eosin staining, immunofluorescence staining of filaggrin, occludin and claudin-1 of mice skin after applying epi-cutaneously PBS as control or 100 µl of a solution containing increasing doses of the papain (0.1µg/µl, 1µg/µl and 10µg/µl). (b) mRNA expression of filaggrin, involucrin, loricrin, keratin-10, claudin-1 and occludin 5 hours after the epicutaneous treatment with different doses of papain.

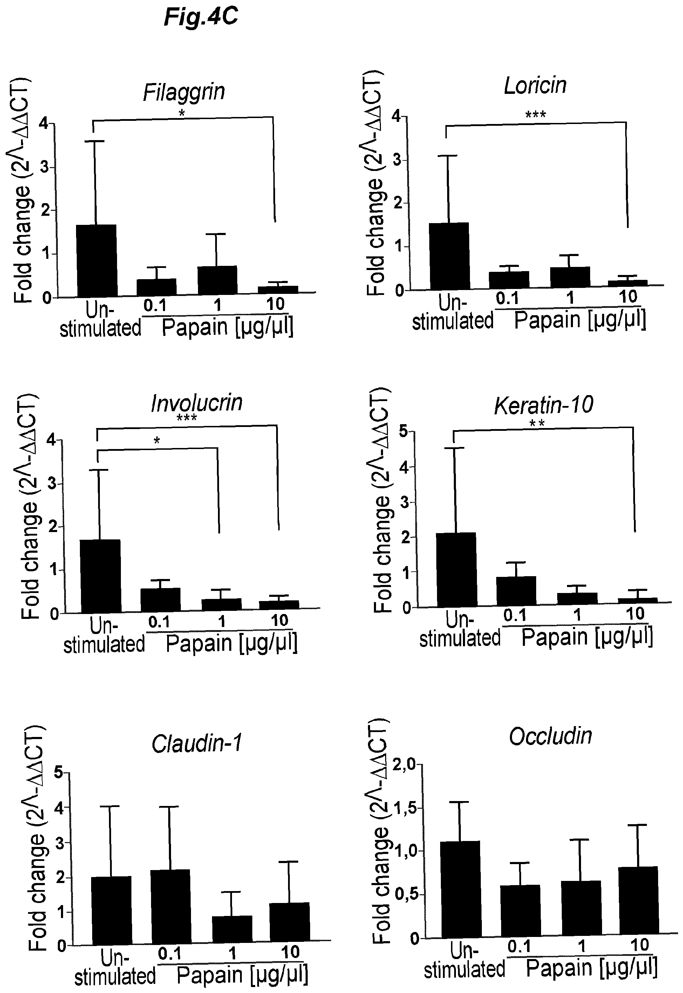

Fig. 4: **Trypsin shows a similar effect on skin epithelial barrier, causing a decrease of EI and an increase of TEWL.** WT C57BL/6 mice were depilated on their back. Three days after depilation, 100 µl of a solution containing trypsin (0.5%), or the cysteine protease papain (10 µg/µl), or PBS as control, by means of a skin patch. EI (a) and TEWL (b) measurements were performed before treatment and 1, 3, 5 and 24h after the treatment. (c) Correlation between EI and TEWL is shown for each time point. Electrical impedance is expressed in kOhm, at the frequency of 1000 Hz. TEWL is expressed in g/m²h. Data are shown as mean ± SD (n=8). *: p<0.05, **: p<0.01.
Fig. 5: **Tape stripping leads to a reduction of EI and an increase of TEWL of the skin.** (a) C57BL/6 nude mice skin was damaged by tape stripping, an efficient approach through which the cell layers of the stratum corneum are successively removed by using adhesive films. EI and TEWL measurements were performed before tape stripping and after 5, 10, 15 and 20 tape strips. (b) Same protocol was followed in human subjects. Electrical impedance is expressed in kOhm, at the frequency of 1000 Hz. TEWL is expressed in g/m²h. Data are shown as mean ± SD, n=12 (a), 5 (b). *: p<0.05, **: p<0.01. ***: p<0.001.
Fig. 6: **Cholera toxin decreases EI of skin ex vivo.** Back skin from C57BL/6 nude mice was isolated and incubated at 37°C for 1h in presence of 2 µg/ml cholera toxin (B), a microbial product which can specifically destroy the epithelial TJs. Electrical impedance is expressed in kOhm, at the frequency of 1000 Hz. Data are shown as mean ± SD, n=5 in A and n=7 in B. *: p<0.05.

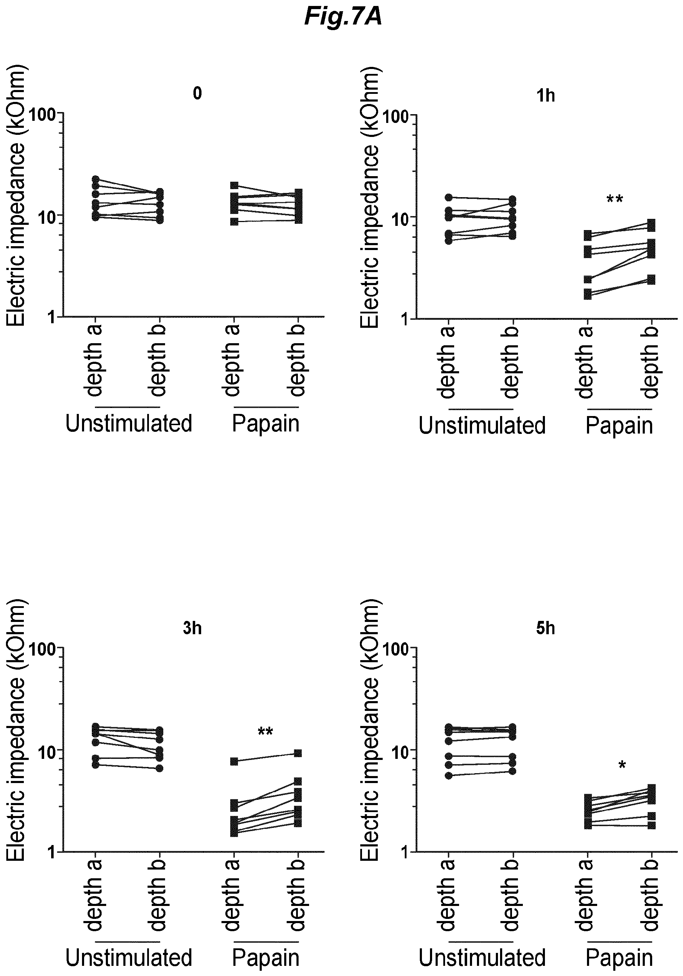

Fig. 7: **Electrical impedance measured at the superficial layer shows a stronger decrease when compared to the deeper layer after papain treatment and tape stripping.** Electrical impedance can be measured at several different depths. Electrical impedance, expressed in kOhm at the frequency of 1000 Hz, is shown at depth a, the most superficial depth, and at depth b, the deepest depth, before the treatment with PBS (control) and papain (10µg/µl) and after 1, 3 and 5 hours from the treatment (a) and before tape stripping and after 5 and 10 tape strips (b). *p<0.05, **: p<0.01.
Fig. 8: **Nyquist plot.** The electrical impedance is a complex number, composed of a real and an imaginary part. A Nyquist Plot is obtained by plotting the real part on the X-axis and the imaginary part on the Y-axis of. Each point of the curve is the impedance at one frequency. Low frequency data are on the right side of the plot and higher frequencies are on the left.
Fig. 9 **AD patients show significantly decreased EI values compared to healthy subjects and their lesions are characterized by a decrease of EI and an increase of TEWL, if compared to non-lesional skin.** EI (a) and TEWL (b) were measured in healthy controls and AD patients in the same body site. In patients measurements were performed both on a lesion and on a non-lesional area close to that lesion. EI is expressed in kOhm, at the frequency of 1000 Hz. TEWL is expressed in g/m2h. *: p<0.05, **: p<0.01,, ****: p<0.0001.
Fig. 10 **Lesional skin shows an increase of EI and a decrease of TEWL during the 21 days treatment.** EI (a) and TEWL (b) were measured in AD patients in the same body site over a time of 21 days, which corresponds to the duration of the treatment at the clinic. EI is expressed in kOhm, at the frequency of 1000 Hz. TEWL is expressed in g/m2h. *: p<0.05, **: p<0.01.

### References

1. Presland RB, Jurevic RJ. Making sense of the epithelial barrier: what molecular biology and genetics tell us about the functions of oral mucosal and epidermal tissues. J Dent Educ. 2002;66(4):564-74.
2. Tamari M, Tanaka S, Hirota T. Genome-wide association studies of allergic diseases. Allergol Int. 2013;62(1):21-8.
3. Tamari M, Hirota T. Genome-wide association studies of atopic dermatitis. J Dermatol. 2014;41(3):213-20.
4. Kubo T, Wawrzyniak P, Morita H, Sugita K, Wanke K, Kast JI, et al. CpG-DNA enhances the tight junction integrity of the bronchial epithelial cell barrier. J Allergy Clin Immunol. 2015;136(5):1413-6.e1-8.
5. Kast JI, Wanke K, Soyka MB, Wawrzyniak P, Akdis D, Kingo K, et al. The broad spectrum of interepithelial junctions in skin and lung. J Allergy Clin Immunol. 2012;130(2):544-7.e4.
6. Wawrzyniak P, Wawrzyniak M, Wanke K, Sokolowska M, Bendelja K, Rückert B, et al. Regulation of bronchial epithelial barrier integrity by type 2 cytokines and histone deacetylases in asthmatic patients. J Allergy Clin Immunol. 2016.
7. Soyka MB, Wawrzyniak P, Eiwegger T, Holzmann D, Treis A, Wanke K, et al. Defective epithelial barrier in chronic rhinosinusitis: the regulation of tight junctions by IFN-γ and IL-4. J Allergy Clin Immunol. 2012;130(5):1087-96.e10.
8. Simon D, Page B, Vogel M, Bussmann C, Blanchard C, Straumann A, et al. Evidence of an abnormal epithelial barrier in active, untreated and corticosteroid-treated eosinophilic esophagitis. Allergy. 2018;73(1):239-47.
9. Mitamura Y, Nunomura S, Nanri Y, Ogawa M, Yoshihara T, Masuoka M, et al. The IL-13/periostin/IL-24 pathway causes epidermal barrier dysfunction in allergic skin inflammation. Allergy. 2018.
10. De Benedetto A, Rafaels NM, McGirt LY, Ivanov AI, Georas SN, Cheadle C, et al. Tight junction defects in patients with atopic dermatitis. J Allergy Clin Immunol. 2011;127(3):773-86.e1-7.
11. Georas SN, Rezaee F. Epithelial barrier function: at the front line of asthma immunology and allergic airway inflammation. J Allergy Clin Immunol. 2014;134(3):509-20.
12. Sandilands A, Sutherland C, Irvine AD, McLean WH. Filaggrin in the frontline: role in skin barrier function and disease. J Cell Sci. 2009;122(Pt 9):1285-94.
13. Furuse M. Molecular Basis of the Core Structure of Tight Junctions. Cold Spring Harbor Perspectives in Biology. 2010;2(1).
14. Ganesan S, Comstock AT, Sajjan US. Barrier function of airway tract epithelium. Tissue Barriers. 2013;1(4):e24997.
15. Zhang N, Van Crombruggen K, Gevaert E, Bachert C. Barrier function of the nasal mucosa in health and type-2 biased airway diseases. Allergy. 2016;71(3):295-307.
16. Schneeberger EE, Lynch RD. The tight junction: a multifunctional complex. Am J Physiol Cell Physiol. 2004;286(6):C1213-28.
17. Shin K, Fogg VC, Margolis B. Tight junctions and cell polarity. Annual review of cell and developmental biology. 2006;22:207-35.
18. Balda MS, Matter K. Tight junctions and the regulation of gene expression. Biochim Biophys Acta. 2009;1788(4):761-7.
19. Assemat E, Bazellieres E, Pallesi-Pocachard E, Le Bivic A, Massey-Harroche D. Polarity complex proteins. Biochim Biophys Acta. 2008;1778(3):614-30.
20. Matter K, Balda MS. SnapShot: Epithelial tight junctions. Cell. 2014;157(4):992- e1.
21. Barbarot S, Auziere S, Gadkari A, Girolomoni G, Puig L, Simpson EL, et al. Epidemiology of atopic dermatitis in adults: Results from an international survey. Allergy. 2018;73(6):1284-93.
22. Nutten S. Atopic dermatitis: global epidemiology and risk factors. Ann Nutr Metab. 2015;66 Suppl 1:8-16.
23. Zaniboni MC, Samorano LP, Orfali RL, Aoki V. Skin barrier in atopic dermatitis: beyond filaggrin. An Bras Dermatol. 2016;91(4):472-8.
24. Soyka MB, Wawrzyniak P, Eiwegger T, Holzmann D, Treis A, Wanke K, et al. Defective epithelial barrier in chronic rhinosinusitis: the regulation of tight junctions by IFN-γ and IL-4. J Allergy Clin Immunol. 2012;130(5):1087-96.e10.
25. Xian M, Wawrzyniak P, Rückert B, Duan S, Meng Y, Sokolowska M, et al. Anionic surfactants and commercial detergents decrease tight junction barrier integrity in human keratinocytes. J Allergy Clin Immunol. 2016;138(3):890-3.e9.
26. Antonov D, Schliemann S, Elsner P. Methods for the Assessment of Barrier Function. Curr Probl Dermatol. 2016;49:61-70.
27. Takai T, Ikeda S. Barrier dysfunction caused by environmental proteases in the pathogenesis of allergic diseases. Allergol Int. 2011;60(1):25-35.

## Claims

1. A medical device (10) for assessing and monitoring epithelial barrier function of a subject in vivo using electrical impedance measurements, said device (10) comprising:
an impedance measuring unit (2) configured to pass an electrical current through the skin of the subject to obtain values of skin impedance of a target tissue region, said impedance measuring unit (2) comprising a plurality of electrodes (14) adapted to be placed in contact with the tissue, wherein each electrode (14) is provided with spikes, thereby forming a spiked surface, or alternatively the electrodes are non-invasive and each electrode has a substantially flat surface adapted to be placed against the tissue of the subject, or a combination of electrodes provided with micro-spikes and non-invasive electrodes, said data comprising at least one impedance value measured in the target tissue region at different tissue layers;
a switching circuit adapted to activate adjacent electrodes (14) in a successive manner to gradually scan tissue of the subject at a first tissue depth so as to obtain a sequence of impedance signals from a selected tissue depth; and
an evaluation unit (4) configured to apply an evaluation procedure for analyzing the epithelial barrier function in the target tissue region on the basis of the measured data set of impedance values for the target tissue region at different tissue layers and to evaluate the obtained data set of impedance values to provide an outcome indicating a status of the epithelial barrier function of the subject; and said device (10) **being characterized in that**
the evaluation unit (4) is configured to use a trained evaluation procedure for analysis of the measured data set of impedance values, wherein said trained evaluation procedure performs:
extracting impedance data from the impedance spectra from obtained data sets of impedance values reflecting tissue characteristics of epithelial barrier function, said extracted impedance data including magnitude information comprising absolute value of magnitude, magnitude gradient, and/or phase information comprising phase angle; and evaluating the obtained data set of impedance to provide the outcome indicating a status of the epithelial barrier function of the subject.

2. The medical device (10) according to claim 1, wherein said evaluation unit (4) is configured to determine atopic dermatitis, AD, of a patient using the evaluation of the obtained data, wherein an impaired skin barrier is a pre-cursor to AD.

3. The medical device (10) according to claim 1, wherein said evaluation unit (4) is configured to determine atopic dermatitis (AD) of a patient using the evaluation of the obtained data, wherein a decrease of impedance compared to non-lesional skin indicates AD.

4. The medical device (10) according to claim 1, wherein said evaluation unit (4) is configured to determine atopic dermatitis, AD, of a patient using the evaluation of the obtained data, wherein a decrease of impedance of over time indicates AD.

5. The medical device (10) according to claim 1 - 4, wherein said evaluation unit (4) is configured to determine a decreased electrical impedance, EI, and increased transepidermal water loss, TEWL, measured at a body site of an AD patient compared to a non-lesional skin site of the AD patient to indicate a lesion at that body site.

6. The medical device (10) according to claim 1 - 5, wherein said evaluation unit (4) is configured to determine a differentiation in electrical impedance, EI, between non-lesional skin of an AD patient and healthy skin of a subject in order to predict AD of subjects.

7. The medical device (10) according to claim 1-6, wherein the electrical impedance measurements are used for determining a patient's response to a drug; and wherein said evaluation unit (4) is configured to determine the patient response of the drug based on status of epithelial barrier function and clinical data including drug prescription.

8. The medical device (10) according to claim 1 - 7, wherein reference data and/or clinical data is used in the evaluation.

## Patentansprüche

1. Medizinische Vorrichtung (10) zur Bewertung und Überwachung der Epithelialbarrierefunktion eines Subjekts in vivo unter Verwendung von elektrischen Impedanzmessungen, wobei die Vorrichtung (10) Folgendes umfasst:
eine Impedanzmesseinheit (2), die so konfiguriert ist, dass sie einen elektrischen Strom durch die Haut des Subjekts leitet, um Werte der Hautimpedanz eines Zielgewebebereichs zu erhalten, wobei die Impedanzmesseinheit (2) eine Vielzahl von Elektroden (14) umfasst, die so ausgebildet sind, dass sie in Kontakt mit dem Gewebe gebracht werden können, wobei jede Elektrode (14) mit Spikes versehen ist, wodurch eine mit Spikes versehene Oberfläche gebildet wird, oder alternativ sind die Elektroden nichtinvasiv und jede Elektrode weist eine im Wesentlichen flache Oberfläche auf, die dazu eingerichtet ist, an das Gewebe des Subjekts angelegt zu werden, oder eine Kombination von mit Mikrospikes versehenen Elektroden und nicht-invasiven Elektroden, wobei die Daten mindestens einen Impedanzwert umfassen, der in dem Zielgewebebereich in verschiedenen Gewebeschichten gemessen wurde;
einen Schaltkreis, der dazu eingerichtet ist, benachbarte Elektroden (14) nacheinander zu aktivieren, um das Gewebe des Subjekts in einer ersten Gewebetiefe allmählich abzutasten, um eine Sequenz von Impedanzsignalen aus einer ausgewählten Gewebetiefe zu erhalten; und
eine Auswertungseinheit (4), die so konfiguriert ist, dass sie ein Auswertungsverfahren zur Analyse der Epithelialbarrierefunktion in dem Zielgewebebereich auf der Grundlage des gemessenen Datensatzes von Impedanzwerten für den Zielgewebebereich in verschiedenen Gewebeschichten ausführt und den erhaltenen Datensatz von Impedanzwerten auswertet, um ein Ergebnis bereitzustellen, das einen Zustand der Epithelialbarrierefunktion des Subjekts angibt; und die Vorrichtung (10) **dadurch gekennzeichnet ist, dass**
die Auswertungseinheit (4) so konfiguriert ist, dass sie ein trainiertes Auswertungsverfahren für die Analyse des gemessenen Datensatzes von Impedanzwerten verwendet, wobei das trainierte Auswerteverfahren Folgendes ausführt:
Extrahieren von Impedanzdaten aus den Impedanzspektren aus erhaltenen Datensätzen von Impedanzwerten, die Gewebeeigenschaften der Epithelialbarrierefunktion wiedergeben, wobei die extrahierten Impedanzdaten Größeninformationen, die den absoluten Wert der Größe, den Größengradienten umfassen, und/oder Phaseninformationen, die den Phasenwinkel umfassen, einschließen; und Auswerten des erhaltenen Impedanzdatensatzes, um ein Ergebnis zu erhalten, das den Zustand der Epithelialbarrierefunktion der Person anzeigt.

2. Medizinische Vorrichtung (10) nach Anspruch 1, wobei die Auswertungseinheit (4) so konfiguriert ist, dass sie anhand der Auswertung der erhaltenen Daten die atopische Dermatitis, AD, eines Patienten bestimmt, wobei eine gestörte Hautbarriere eine Vorstufe von AD ist.

3. Medizinische Vorrichtung (10) nach Anspruch 1, wobei die Auswertungseinheit (4) so konfiguriert ist, dass sie anhand der Auswertung der erhaltenen Daten die atopische Dermatitis (AD) eines Patienten bestimmt, wobei eine Abnahme der Impedanz im Vergleich zu nichtläsionaler Haut auf AD hinweist.

4. Medizinische Vorrichtung (10) nach Anspruch 1, wobei die Auswertungseinheit (4) so konfiguriert ist, dass sie anhand der Auswertung der erhaltenen Daten die atopische Dermatitis, AD, eines Patienten bestimmt, wobei eine Abnahme der Impedanz im Laufe der Zeit auf AD hinweist.

5. Medizinische Vorrichtung (10) nach Anspruch 1-4, wobei die Auswertungseinheit (4) so konfiguriert ist, dass sie eine verringerte elektrische Impedanz (EI) und einen erhöhten transepidermalen Wasserverlust (TEWL), gemessen an einer Körperstelle eines AD-Patienten im Vergleich zu einer nichtläsionalen Hautstelle des AD-Patienten, bestimmt, um eine Läsion an dieser Körperstelle zu kennzeichnen.

6. Medizinische Vorrichtung (10) nach einem der Ansprüche 1-5, wobei die Auswertungseinheit (4) so konfiguriert ist, dass sie einen Unterschied in der elektrischen Impedanz, EI, zwischen der nichtläsionalen Haut eines AD-Patienten und der gesunden Haut einer Person bestimmt, um AD bei Subjekten vorherzusagen.

7. Medizinische Vorrichtung (10) nach einem der Ansprüche 1-6, wobei die Messungen der elektrischen Impedanz zur Bestimmung der Reaktion eines Patienten auf ein Medikament verwendet werden; und wobei die Auswertungseinheit (4) so konfiguriert ist, dass sie die Reaktion des Patienten auf das Medikament auf der Grundlage des Zustands der Epithelialbarrierefunktion und der klinischen Daten einschließlich der Medikamentenverschreibung bestimmt.

8. Medizinische Vorrichtung (10) nach einem der Ansprüche 1-7, wobei für die Bewertung Referenzdaten und/oder klinische Daten verwendet werden.

## Revendications

1. Dispositif (10) médical pour évaluer et surveiller la fonction de barrière épithéliale d'un sujet in vivo à l'aide de mesures d'impédance électrique, ledit dispositif (10) comprenant :
une unité (2) de mesure d'impédance configurée pour faire passer un courant électrique à travers la peau du sujet pour obtenir des valeurs d'impédance cutanée d'une région tissulaire cible, ladite unité (2) de mesure d'impédance comprenant une pluralité d'électrodes (14) adaptées pour être mises en contact avec le tissu, dans lequel chaque électrode (14) est pourvue de pointes, formant ainsi une surface à pointes, ou en variante les électrodes sont non invasives et chaque électrode présente une surface sensiblement plane adaptée pour être placée contre le tissu du sujet, ou une combinaison d'électrodes munies de micropointes et d'électrodes non invasives, lesdites données comprenant au moins une valeur d'impédance mesurée dans la région tissulaire cible à différentes couches tissulaires ;
un circuit de commutation adapté pour activer des électrodes (14) adjacentes de manière successive pour balayer progressivement le tissu du sujet à une première profondeur tissulaire de manière à obtenir une séquence de signaux d'impédance à partir d'une profondeur tissulaire sélectionnée ; et
une unité d'évaluation (4) configurée pour appliquer une procédure d'évaluation afin d'analyser la fonction de barrière épithéliale dans la région tissulaire cible sur la base de l'ensemble de données mesurées de valeurs d'impédance pour la région tissulaire cible à différentes couches tissulaires et pour évaluer l'ensemble de données obtenues de valeurs d'impédance pour fournir un résultat indiquant un état de la fonction de barrière épithéliale du sujet ; et ledit dispositif (10) **étant caractérisé en ce que**
l'unité d'évaluation (4) est configurée pour utiliser une procédure d'évaluation entraînée pour l'analyse de l'ensemble de données mesurées de valeurs d'impédance, dans lequel ladite procédure d'évaluation entraînée effectue :
l'extraction de données d'impédance à partir des spectres d'impédance à partir d'ensembles de données obtenues de valeurs d'impédance reflétant les caractéristiques tissulaires de la fonction de barrière épithéliale, lesdites données d'impédance extraites incluant des informations de grandeur comprenant une valeur absolue de grandeur, un gradient de grandeur et/ou des informations de phase comprenant un angle de phase ; et l'évaluation de l'ensemble de données obtenues d'impédance pour fournir le résultat indiquant un état de la fonction de barrière épithéliale du sujet.

2. Dispositif (10) médical selon la revendication 1, dans lequel ladite unité d'évaluation (4) est configurée pour déterminer la dermatite atopique, AD, d'un patient à l'aide de l'évaluation des données obtenues, dans lequel une barrière cutanée altérée est un précurseur d'AD.

3. Dispositif (10) médical selon la revendication 1, dans lequel ladite unité d'évaluation (4) est configurée pour déterminer une dermatite atopique (AD) d'un patient à l'aide de l'évaluation des données obtenues, dans lequel une diminution de l'impédance par rapport à une peau non lésionnelle indique une AD.

4. Dispositif (10) médical selon la revendication 1, dans lequel ladite unité d'évaluation (4) est configurée pour déterminer la dermatite atopique, AD, d'un patient à l'aide de l'évaluation des données obtenues, dans lequel une diminution de l'impédance au fil du temps indique une AD.

5. Dispositif (10) médical selon la revendication 1 à 4, dans lequel ladite unité d'évaluation (4) est configurée pour déterminer une diminution de l'impédance électrique, El, et une augmentation de la perte d'eau transépidermique, TEWL, mesurées au niveau d'un site corporel d'un patient atteint d'AD par rapport à un site cutané non lésionnel du patient atteint d'AD pour indiquer une lésion au niveau de ce site corporel.

6. Dispositif (10) médical selon les revendications 1 à 5, dans lequel ladite unité d'évaluation (4) est configurée pour déterminer une différenciation d'impédance électrique, El, entre la peau non lésionnelle d'un patient atteint d'AD et la peau saine d'un sujet afin de prédire l'AD chez des sujets.

7. Dispositif (10) médical selon les revendications 1 à 6, dans lequel les mesures d'impédance électrique sont utilisées pour déterminer la réponse d'un patient à un médicament ; et dans lequel ladite unité d'évaluation (4) est configurée pour déterminer la réponse du patient au médicament sur la base de l'état de la fonction de barrière épithéliale et de données cliniques incluant la prescription de médicaments.

8. Dispositif (10) médical selon les revendications 1 à 7, dans lequel des données de référence et/ou des données cliniques sont utilisées dans l'évaluation.
